(19) Europäisches Patentamt / European Patent Office / Office européen des brevets

(11) **EP 2 960 703 A1**

(12) **EUROPEAN PATENT APPLICATION**
published in accordance with Art. 153(4) EPC

(43) Date of publication:
**30.12.2015 Bulletin 2015/53**

(51) Int Cl.:
**G02B 21/06** (2006.01)  **A61B 1/00** (2006.01)
**G02B 23/24** (2006.01)

(21) Application number: **14754832.5**

(22) Date of filing: **25.02.2014**

(86) International application number:
**PCT/JP2014/054513**

(87) International publication number:
**WO 2014/129650 (28.08.2014 Gazette 2014/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.02.2013 JP 2013034592**

(71) Applicant: **Olympus Corporation**
**Shibuya-ku**
**Tokyo 151-0072 (JP)**

(72) Inventors:
• **IGA, Yasunobu**
**Tokyo 151-0072 (JP)**
• **TAKAHASHI, Shintaro**
**Tokyo 151-0072 (JP)**

(74) Representative: **Schicker, Silvia**
**Wuesthoff & Wuesthoff**
**Patentanwälte PartG mbB**
**Schweigerstraße 2**
**81541 München (DE)**

(54) **BEAM SPLITTER DEVICE, SCANNING OBSERVATION APPARATUS, LASER SCANNING MICROSCOPE, AND LASER SCANNING ENDOSCOPE**

(57)     A beam splitter apparatus (1) of the present invention is provided with demultiplexing portions (13a and 13b) that split an input pulsed light beam (L0) into a plurality of optical paths (11a, 11b, 12a, and 12b); relay optical systems (141a to 144a and 141b to 144b) that individually relay the pulsed light beams in the plurality of optical paths; a multiplexing portion (13c) that multiplexes the plurality of pulsed light beams (L1 to L4) that have been relayed through the optical paths; and delaying portions (12a and 12b) and divergence-angle setting portions (151a and 151b and 152a and 152b) that respectively give the pulsed light beams, which are individually guided through the plurality of optical paths, relative time delays that are large enough to separate the signal lights (LS) and divergence angles that are different from each other.

FIG. 4

**Description**

{Technical Field}

The present invention relates to a beam splitter apparatus, a scanning observation apparatus, a laser-scanning microscope, and a laser-scanning endoscope.

{Background Art}

In the related art, there are known scanning microscopes that acquire an image of a specimen by two-dimensionally scanning a beam over the specimen (for example, see Patent Literature 1). According to Patent Literature 1, it is possible to change an image-acquisition region in the depth direction of the specimen by moving the focal point of the beam also in the optical-axis direction by using a wavefront converting device.

{Citation List}

{Patent Literature}

{PTL 1} Japanese Unexamined Patent Application, Publication No. 2004-109219

{Summary of Invention}

{Technical Problem}

The lifetime of fluorescence generated by molecular reactions in a biological subject is assumed to be about three nanoseconds. Therefore, in order to observe reactions occurring at different depths in the biological subject at substantially same timescale as the fluorescence lifetime by using the device disclosed in Patent Literature 1, the beam needs to be modulated at a high speed of several hundreds of megahertz. However, because the wavefront converting device moves the position of the focal point of the beam by mechanically changing the shape of a reflecting surface thereof, there is a limit to enhancing the speed at which the focal point is moved, and thus, in principle, it is difficult to achieve the above-described high-speed modulation. Therefore, with the device disclosed in Patent Literature 1, there is a problem in that it is not possible to observe reactions occurring at different depths in the specimen at substantially the same point in time.

The present invention has been conceived in light of the above-described circumstances, and an object thereof is to provide a beam splitter apparatus, a scanning observation apparatus, a laser-scanning microscope, and a laser-scanning endoscope with which it is possible to observe even reactions occurring at different depths in a specimen at the same point in time.

{Solution to Problem}

In order to achieve the above-described object, the present invention provides the following solutions.

A first aspect of the present invention is a beam splitter apparatus that is applied to an observation apparatus that irradiates a specimen with pulsed light beams to induce a molecular reaction in the specimen and that observes signal lights generated by this reaction, the beam splitter apparatus including a demultiplexing portion that splits an input pulsed light beam into a plurality of optical paths; relay optical systems that are provided in the plurality of optical paths and that relay the pulsed light beams guided through the individual optical paths; a multiplexing portion that multiplexes the plurality of the pulsed light beams that have been relayed through the individual optical paths by the relay optical systems; delaying portions that are provided in the plurality of optical paths and that give the pulsed light beams, which are guided through the individual optical paths, relative time delays that are large enough to separate the plurality of the signal lights from each other; and divergence-angle setting portions that are provided in the plurality of optical paths and that give the pulsed light beams that are guided through the individual optical paths divergence angles that are different from each other.

With the first aspect of the present invention, the pulsed light beam entering from outside is split by the demultiplexing portion, after which the split beams are individually given time delays and divergence angles while being relayed by the relay optical systems along the individual optical paths, and are multiplexed by the multiplexing portion. Therefore, the plurality of pulsed light beams, whose focal points are formed at different positions in the optical-axis direction, exit the multiplexing portion with sufficiently short time intervals that correspond to the time delays. Specifically, the individual pulsed light beams are substantially simultaneously radiated onto different depth positions in the specimen. By doing so, it is possible to observe reactions occurring at different depths in a specimen at the same point in time.

In the first aspect described above, the relay optical systems may be provided with pairs of lenses, and each of the divergence-angle setting portions may be disposed between the pair of lenses.

By doing so, beam divergence angles achieved at the pair of lenses and thereafter are changed by changing the beam focusing positions between the pair of lenses in the optical-axis direction. By doing so, it is possible to change the beam divergence angles in a continuous manner.

In the first aspect described above, the divergence-angle setting portions may be provided with mirror pairs that deflect the pulsed light beams split by the demultiplexing portion toward the multiplexing portion so as to trace out a rectangular shape, and the mirror pairs may be provided in such a way that the mirror pairs can be moved together in a direction parallel to entrance optical axes of the pulsed light beams coming from the demultiplexing portion.

By doing so, it is possible to change the focal-point positions of the pulsed light beams in the optical-axis direction by moving the mirror pairs in the direction parallel to the entrance optical axes.

In the first aspect described above, the mirror pairs may be provided in such a way that the mirror pairs can be moved together in a direction that intersects the entrance optical axes of the pulsed light beams coming from the demultiplexing portion.

By doing so, it is possible to change the focal-point positions the plurality of the pulsed light beams in the direction that intersects the optical axes by moving the mirror pairs in the direction that intersects the entrance optical axes.

In the first aspect described above, the divergence-angle setting portions may be provided with focusing lenses that are provided in the plurality of optical paths and that convert the pulsed light beams that are guided through the individual optical paths to converging beams that are converged at positions differing from each other in the optical-axis direction; and a collimator lens that is provided behind the multiplexing portion and that receives all of the pulsed light beams that have been converted to the converging beams by the individual focusing lenses and that converts the received beams to substantially collimated beams having different divergence angles from each other. In this case, the focusing lenses may be provided so as to be movable in the optical-axis direction.

By doing so, it is possible to shift the focal-point positions in the optical-axis direction without changing the respective time-delay levels of the plurality of pulsed light beams.

In the first aspect described above, the individual focusing lenses may be provided so as to be movable in the optical-axis direction in synchronization with each other.

By doing so, by moving the plurality of focusing lenses at the same time, the focal-point positions of the plurality of pulsed light beams are moved together in the optical-axis direction. By doing so, it is possible to scan the plurality of pulsed light beams at irradiation positions located in the depth direction of the specimen.

The first aspect described above may be provided with light-intensity adjusting portions that are provided in the plurality of optical paths and that adjust intensities of the pulsed light beams that are guided along the individual optical paths.

By doing so, it is possible to compensate for the variability of the intensities of the signal lights generated at the specimen caused by the fact that the specimen is irradiated with the plurality of pulsed light beams at positions that differ in the depth direction.

In the first aspect described above, at least one of the demultiplexing portion and the multiplexing portion may be provided with a polarizing beam splitter, and the light-intensity adjusting portions may be provided with a polarization adjusting portion that is provided in front of the polarizing beam splitter and that adjusts the polarization of the pulsed light beams.

By doing so, because the splitting ratio of the polarizing beam splitter depends on the polarization of the pulsed light beams, it is possible to adjust the intensities of the individual pulsed light beams by adjusting the polarization of the pulsed light beams that enter the polarizing beam splitter by using the polarization adjusting portion.

In the first aspect described above, the light-intensity adjusting portions may be provided with parallel plates that are provided in the plurality of optical paths and with which transmittances of the pulsed light beams can be changed.

By doing so, the intensities of the individual pulsed light beams can easily be adjusted.

In the first aspect described above, multiple sets of the demultiplexing portion, the relay optical systems, the multiplexing portion, the delaying portions, and the divergence-angle setting portions may be provided in series.

By doing so, it is possible to double the number of pulsed light beams finally generated from a single pulsed light beam.

A second aspect of the present invention is a scanning observation apparatus including any one of the beam splitter apparatuses described above; a scanning portion that scans a plurality of pulsed light beams emitted from the beam splitter apparatus in a direction that intersects the optical axes; an observation optical system that irradiates the specimen with the pulsed light beams scanned by the scanning portion; and a detection system that detects the signal lights coming from the specimen.

With the second aspect of the present invention, the signal lights generated at the specimen are detected by the detection system when the plurality of pulsed light beams that have exited the beam splitter apparatus are radiated onto the specimen by means of the observation optical system while being scanned by the scanning portion. In this case, because the individual pulsed light beams are substantially simultaneously scanned in a synchronized manner on the observation planes at different depths in the specimen, it is possible to observe even reactions occurring at different depths in the specimen at the same point in time.

With the second aspect described above, it is preferable that the observation optical system be provided with an objective lens that focuses the pulsed light beams on the specimen.

By doing so, it is possible to efficiently generate single light beams by using the individual pulsed light beams.

The second aspect described above may be provided with a control portion that synchronizes a timing at which the detection system detects the signal lights with the pulsed light beams; a restoring portion that restores two-dimensional information or three-dimensional information by associating the signal lights detected by the detection system and the scanning positions of the pulsed light beams on the specimen; and a display portion that displays the two-dimensional information or the three-dimensional information that has been restored by the restoring portion.

By doing so, it is possible to obtain a two-dimensional image or a three-dimensional image of the detected signal lights by detecting the signal lights generated by irradiation with the pulsed light beams at an appropriate timing.

A third aspect of the present invention is a laser-scanning microscope including any one of the scanning observation apparatuses described above; and a laser light source that supplies the beam splitter apparatus with pulsed laser beams that serve as the pulsed light beams.

A fourth aspect of the present invention is a laser-scanning endoscope including any one of the scanning observation apparatuses described above.

{Advantageous Effects of Invention}

The present invention affords an advantage in that it is possible to observe even reactions occurring at different depths in a specimen at the same point in time.

{Brief Description of Drawings}

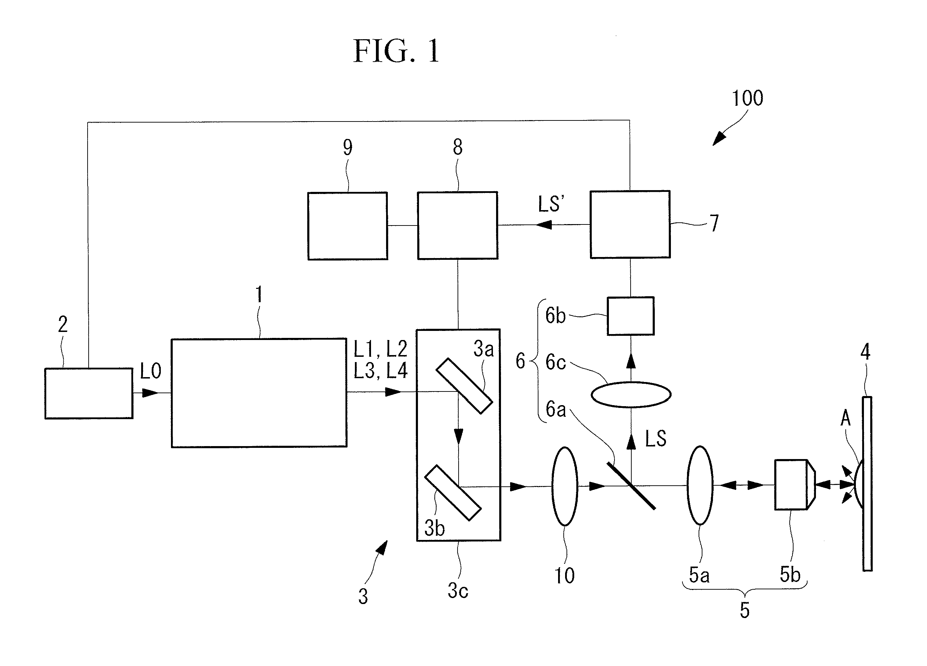

{Fig. 1} Fig. 1 is an overall configuration diagram showing a laser-scanning microscope according to a first embodiment of the present invention.

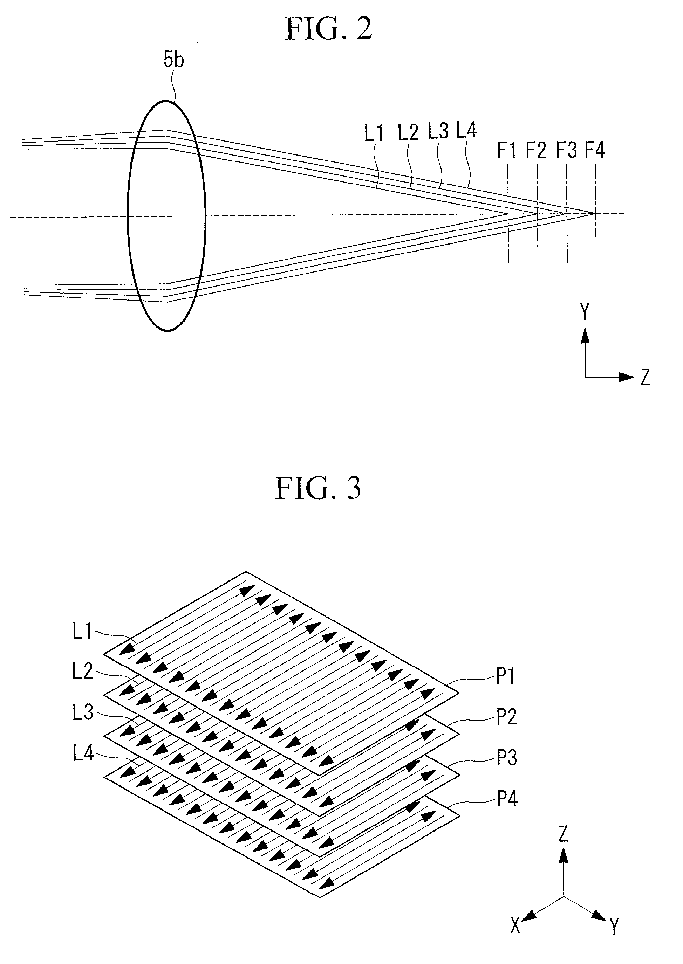

{Fig. 2} Fig. 2 is a diagram for explaining, for the laser-scanning microscope in Fig. 1, focal-point positions of four pulsed laser beams radiated onto a specimen.

{Fig. 3} Fig. 3 is a diagram for explaining observation planes to be scanned with the four pulsed laser beams in the laser-scanning microscope in Fig. 1.

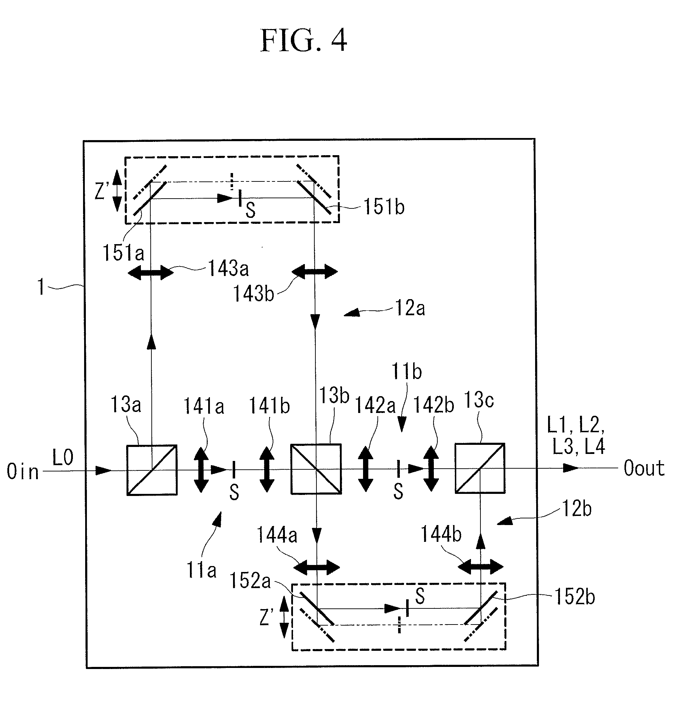

{Fig. 4} Fig. 4 is an overall configuration diagram showing a beam splitter apparatus according to the first embodiment of the present invention.

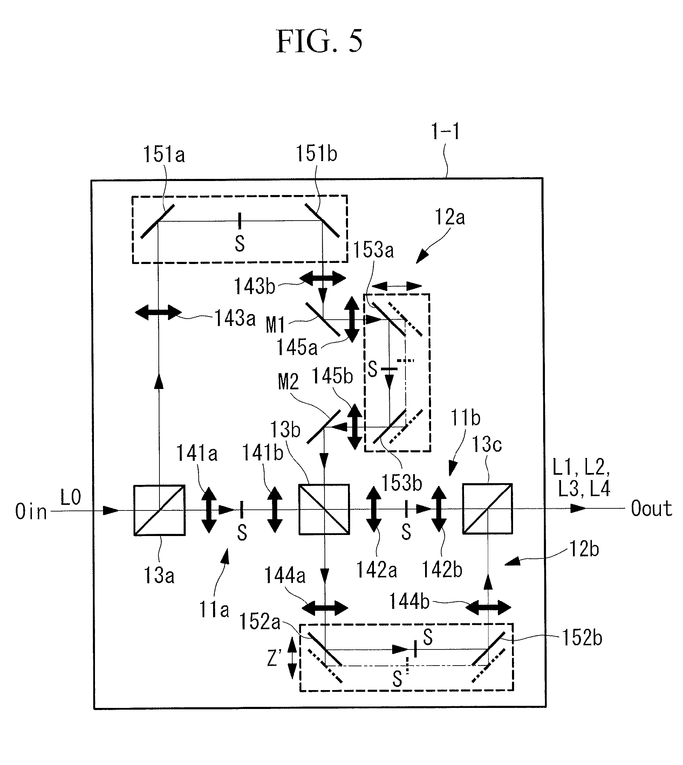

{Fig. 5} Fig. 5 is an overall configuration diagram showing a first modification of the beam splitter apparatus in Fig. 4.

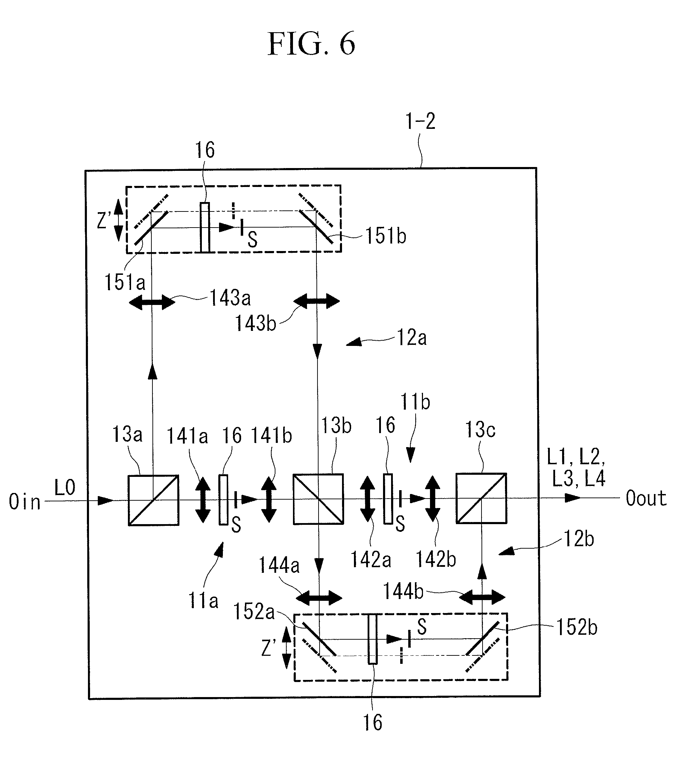

{Fig. 6} Fig. 6 is an overall configuration diagram showing a second modification of the beam splitter apparatus in Fig. 4.

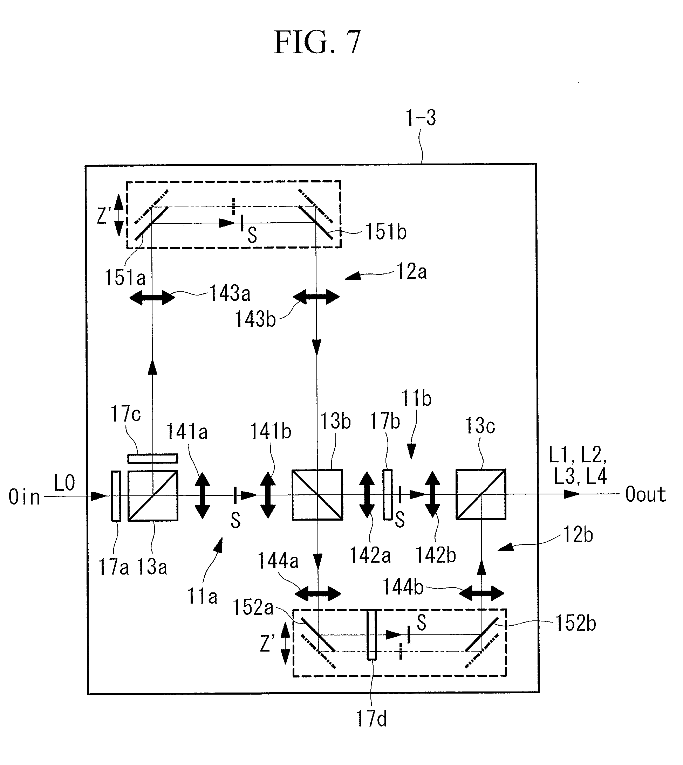

{Fig. 7} Fig. 7 is an overall configuration diagram showing a third modification of the beam splitter apparatus in Fig. 4.

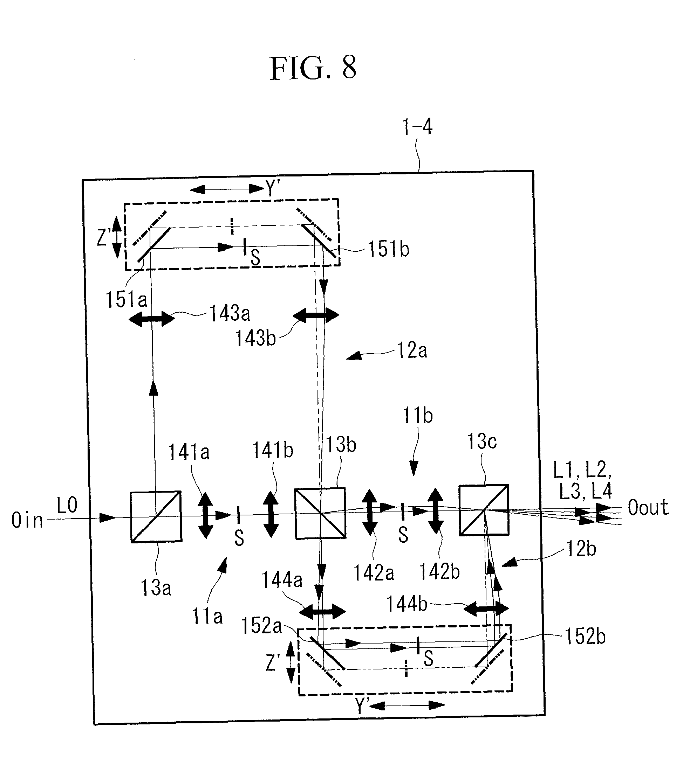

{Fig. 8} Fig. 8 is an overall configuration diagram showing a fourth modification of the beam splitter apparatus in Fig. 4.

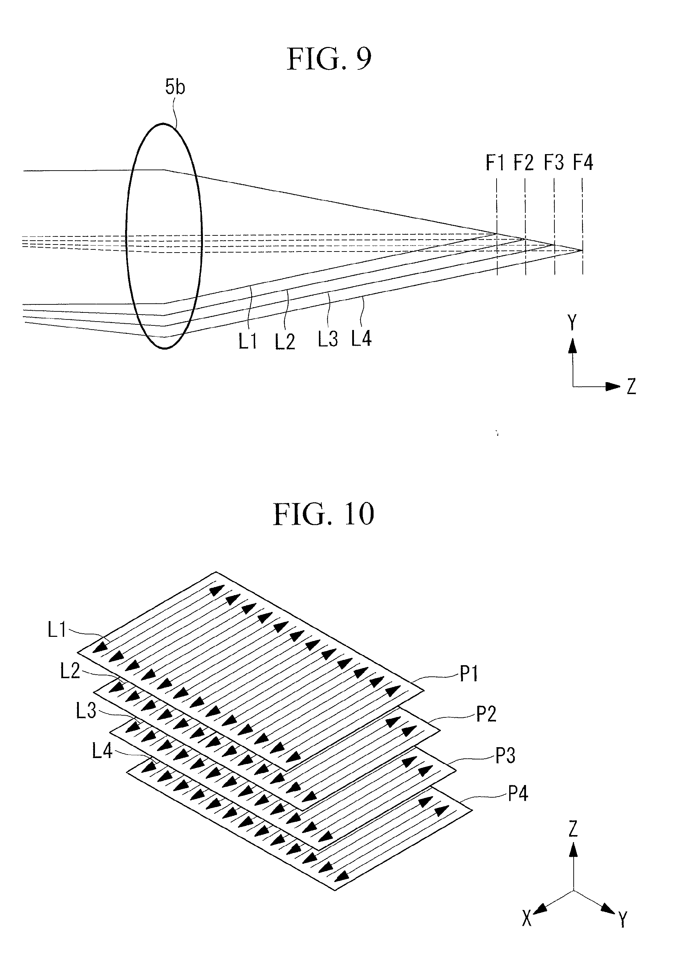

{Fig. 9} Fig. 9 is a diagram for explaining focal-point positions of four pulsed laser beams generated by the beam splitter apparatus in Fig. 8.

{Fig. 10} Fig. 10 is a diagram for explaining observation planes to be scanned with the four pulsed laser beams generated by the beam splitter apparatus in Fig. 8.

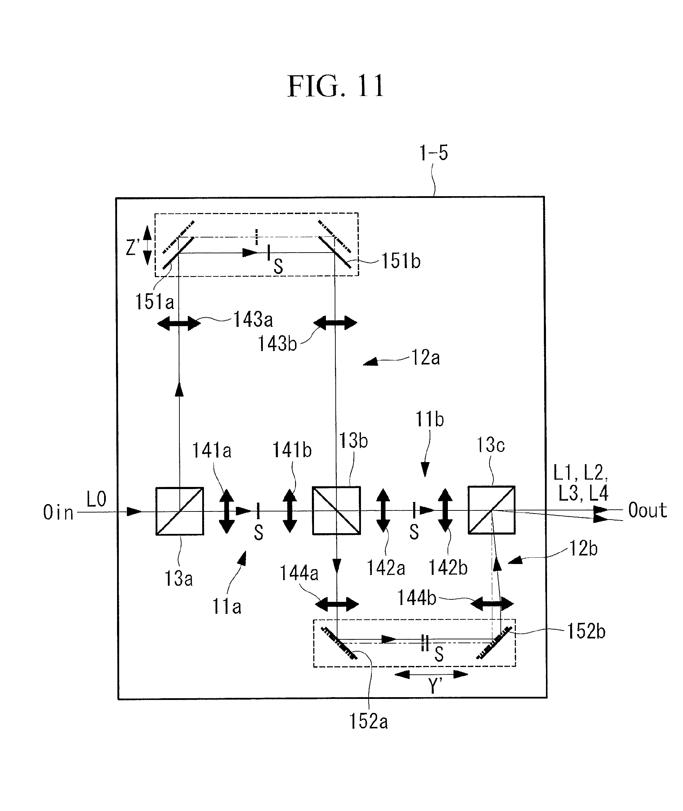

{Fig. 11} Fig. 11 is an overall configuration diagram showing a fifth modification of the beam splitter apparatus in Fig. 4.

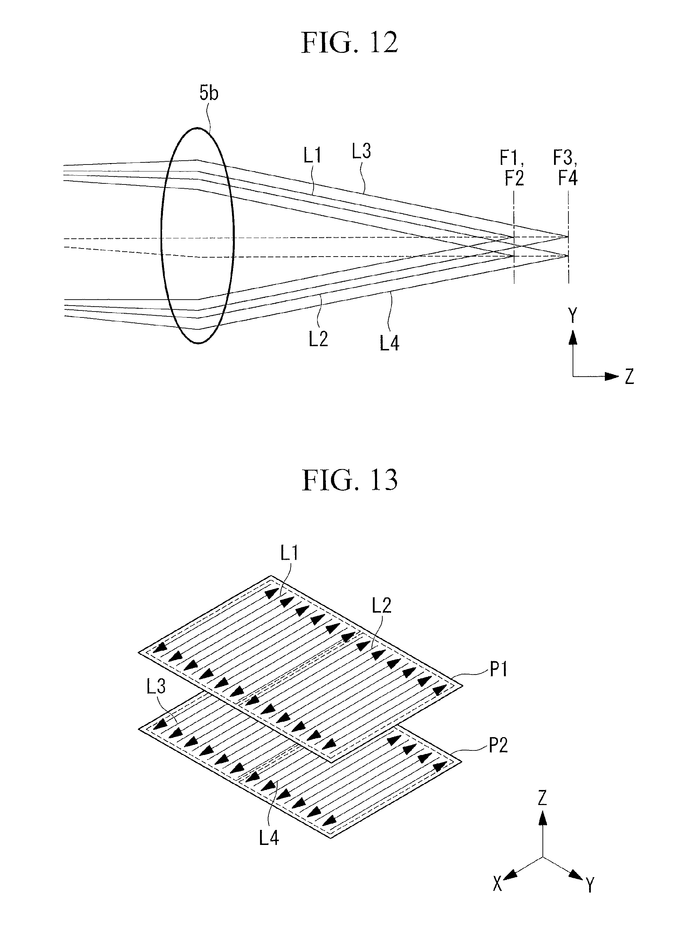

{Fig. 12} Fig. 12 is a diagram for explaining focal-point positions of four pulsed laser beams generated by the beam splitter apparatus in Fig. 11.

{Fig. 13} Fig. 13 is a diagram for explaining observation planes to be scanned with the four pulsed laser beams generated by the beam splitter apparatus in Fig. 11.

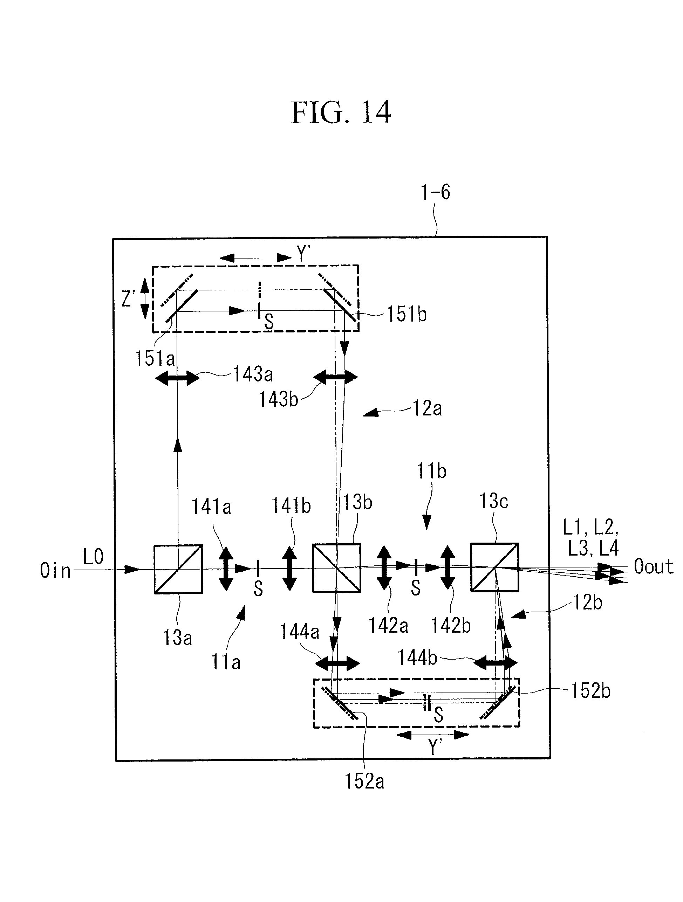

{Fig. 14} Fig. 14 is an overall configuration diagram showing a sixth modification of the beam splitter apparatus in Fig. 4.

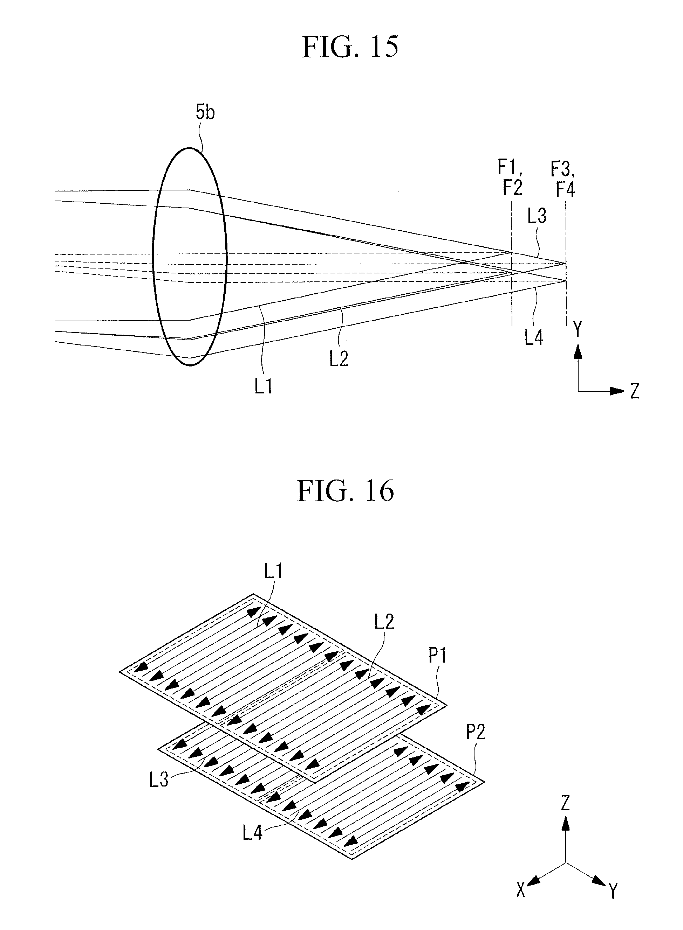

{Fig. 15} Fig. 15 is a diagram for explaining focal-point positions of four pulsed laser beams generated by the beam splitter apparatus in Fig. 14.

{Fig. 16} Fig. 16 is a diagram for explaining observation planes to be scanned with the four pulsed laser beams generated by the beam splitter apparatus in Fig. 14.

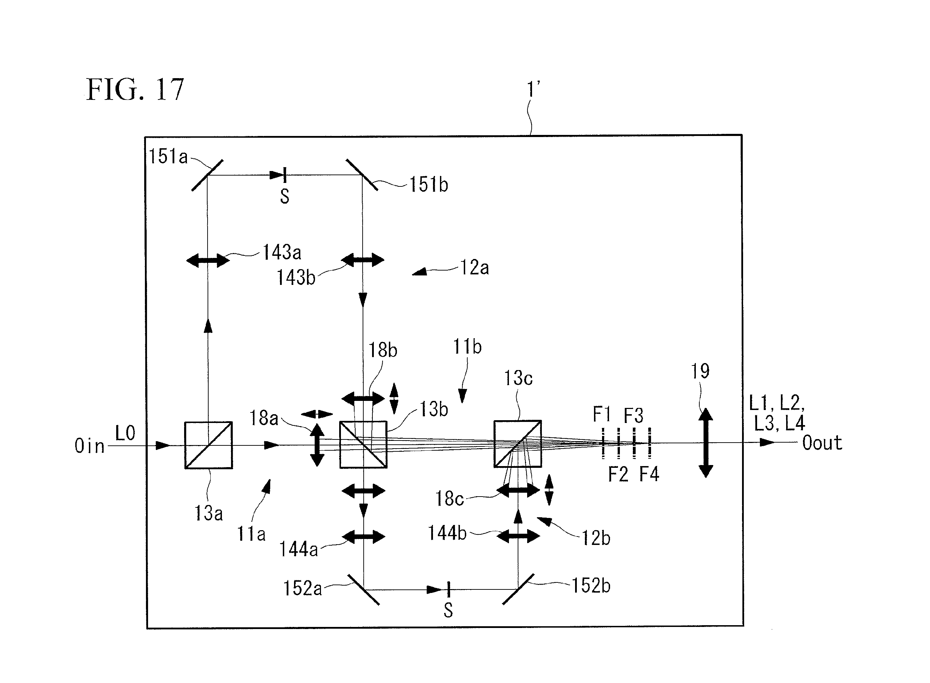

{Fig. 17} Fig. 17 is an overall configuration diagram showing a beam splitter apparatus according to a second embodiment of the present invention.

{Description of Embodiments}

{First Embodiment}

A beam splitter apparatus 1 according to a first embodiment of the present invention and a laser-scanning microscope 100 provided with the same will be described below with reference to Figs. 1 to 16.

First, the overall configuration of the laser-scanning microscope 100 will be described.

As shown in Fig. 1, the laser-scanning microscope 100 according to this embodiment is provided with a laser light source 2 that emits a pulsed laser beam (pulsed light beam) L0, a beam splitter apparatus 1 that generates four pulsed laser beams L1 to L4 from the pulsed laser beam L0 emitted from the laser light source 2, a scanning portion 3 that scans the four pulsed laser beams L1 to L4 emitted from the beam splitter apparatus 1 in the direction that intersects the optical axis, a stage 4 that supports a specimen A, an observation optical system 5 that radiates the pulsed laser beams L1 to L4 coming from the scanning portion 3 onto the specimen A, a detection system 6 that detects a signal light LS that is generated at the specimen A due to the irradiation with the pulsed laser beams L1 to L4, a control portion 7 that generates a detection signal LS' by synchronizing the operation of the laser light source 2 and that of a detecting portion 6b provided in the detection system 6, a restoring portion 8 that creates an image of the specimen A based on the detection signal LS' generated by the control portion 7 and scanning position information from a mirror driving portion 3c provided in the scanning portion 3, and a display portion 9 that displays the image created by the restoring portion 8. Reference sign 10 indicates a pupil projection lens that projects a plane that is optically conjugate with a pupil of an objective lens 5b provided in the observation optical system 5 onto the scanning portion 3.

The laser light source 2 emits the pulsed laser beam L0 that induces a reaction, for example, photoemission, of specific molecules contained in the specimen A.

As will be described later in detail, the beam splitter apparatus 1 generates the four pulsed laser beams L1 to L4 by dividing the ray bundle of the single pulsed laser beam L0, which has entered the beam splitter apparatus 1 by coming from the laser light source 2, into a plurality of beams, and individually applies different delay times and divergence angles to the four generated pulsed laser beams L1 to L4. By doing so, as shown in Fig. 2, the four pulsed laser beams L1 to L4, whose focal points are formed at different positions in the optical-axis direction when converged by the objective lens 5b (to be described later), are sequentially emitted with sufficiently short time intervals therebetween.

The scanning portion 3 is provided with two mirrors 3a and 3b that are rotatable about two mutually perpendicular axes. The scanning portion 3 is configured so as to perform raster scanning of the pulsed laser beams L1 to L4 in a plane that intersects the optical axis by appropriately changing the direction in which the pulsed laser beams L1 to L4 are reflected by controlling the rotational angles of the two mirrors 3a and 3b. A plane that is optically conjugate with the pupil of the objective lens 5b is positioned on the reflecting surfaces of the mirrors 3a and 3b or between the mirrors 3a and 3b. A lens pair that relays the pupil may be disposed between the beam splitter apparatus 1 and the mirror 3a as needed.

The observation optical system 5 is provided with an imaging lens 5a that forms an image by using the pulsed laser beams L1 to L4 that have passed through the pupil projection lens 10 and an objective lens 5b that makes the pulsed laser beams L1 to L4 with which an image is formed by the imaging lens 5a converge on the specimen A.

The detection system 6 is provided with a dichroic mirror 6a that reflects, among the beams that have been made to converge by the observation optical system 5, only the signal light LS, a detecting portion 6b that detects the signal light LS reflected by the dichroic mirror 6a, and a detection lens 6c that focuses the signal light LS at an photoreceiver of the detecting portion 6b.

The control portion 7 synchronizes the timing at which the signal light LS is detected by the detecting portion 6b with the timing at which the pulsed laser beam L0 is emitted from the laser light source 2.

The restoring portion 8 restores two-dimensional information or three-dimensional information by associating the signal light LS detected by the detecting portion 6b with the scanning positions of the pulsed laser beams L1 to L4, and outputs the restored two-dimensional information or three-dimensional information to the display portion 9.

Next, the operation of the thus-configured laser-scanning microscope 100 will be described.

The pulsed laser beam L0 emitted from the laser light source 2 is converted to the four pulsed laser beams L1 to L4 at the beam splitter apparatus 1, and these are subsequently made to enter the observation optical system 5 via the scanning portion 3, are radiated onto the specimen A from the observation optical system 5, and are used to perform raster scanning over the specimen A.

Regarding the signal light LS, such as fluorescence, generated at the specimen A due to the irradiation with the pulsed laser beams L1 to L4, the light-detecting portion 6b detects the signal light LS and converts it to an electrical signal corresponding to the intensity thereof, the restoring portion 8 associates the signal with the position on the specimen A, thus generating an image, and the generated two-dimensional image or three-dimensional image is displayed on the display portion 9. At this time, as shown in Fig. 3, the four pulsed laser beams L1 to L4 generated by the beam splitter apparatus 1 are substantially simultaneously scanned in a synchronized manner on four observation planes P1 to P4 whose positions in the depth direction (Z-direction) are different. Therefore, at the restoring portion 8, it is possible to substantially simultaneously generate four two-dimensional images or three-dimensional images that show different depths in the specimen A.

Next, the beam splitter apparatus 1 according to this embodiment will be described.

As shown in Fig. 4, the beam splitter apparatus 1 according to this embodiment is provided with main optical path 11a and 11b that is provided on an entrance optical axis $O_{in}$ so as to form a straight line and two delay optical paths (delaying portions) 12a and 12b that split off from the main optical path 11a and 11b. The main optical path 11a and 11b and the two delay optical paths 12a and 12b have different optical path lengths from each other, where the first delay optical path 12a has an optical path length that is longer than the optical path length of the main optical path 11a and 11b by a length 2d, and the second delay optical path 12b has an optical path length that is longer than the optical path length of the main optical path 11a and 11b by a length d.

In the main optical path 11a and 11b, a first beam splitter (demultiplexing portion) 13a, a second beam splitter (demultiplexing portion) 13b, and a third beam splitter (multiplexing portion) 13c are provided in series in this order from the entrance side. The single pulsed laser beam L0 is divided twice by the first beam splitter 13a and the second beam splitter 13b, and thus, the four pulsed laser beams L1 to L4 that have travelled along the different optical paths 11a, 11b, 12a, and 12b join at the third beam splitter 13c so as to be emitted therefrom along an exit optical axis $O_{out}$, which is an extension of the entrance optical axis $O_{in}$. In the following, of the main optical path, the portion between the first beam splitter 13a and the second beam splitter 13b will be referred to as a first main optical path 11a, and a portion between the second beam splitter 13b and the third beam splitter 13c will be referred to as a second main optical path 11b.

Specifically, the first beam splitter 13a divides the pulsed laser beam L0 into two beams, one of which is reflected into the first delay optical path 12a, and the other beam passes straight through into the first main optical path 11a. The second beam splitter 13b divides the pulsed laser beam that comes thereinto via the first main optical path 11a into two beams, one of which is reflected into the second delay optical path 12b, and the other beam passes straight through into the third beam splitter 13c. Furthermore, the second beam splitter 13b divides the pulsed laser beam that comes thereinto via the first delay optical path 12a into two beams, one of which is reflected into the second delay optical path 12b, and the other beam passes straight through into the third beam splitter 13c.

By doing so, the pulsed laser beam L1 that has passed through the first main optical path 11a and the second main optical path 11b, the pulsed laser beam L2 that has passed through the first main optical path 11a and the second delay optical path 12b, the pulsed laser beam L3 that has passed through the first delay optical path 12a and the second main optical path 11b, and the pulsed laser beam L4 that has passed through the first delay optical path 12a and the second delay optical path 12b join at the third beam splitter 13c. The third beam splitter allows the pulsed laser beams that have travelled along the second main optical path 11b to pass therethrough and reflects the pulsed laser beams that have travelled along the second delay optical path 12b, thus emitting the four pulsed laser beams L1 to L4 along the single exit optical axis $O_{out}$.

Note that, in the optical-path configuration shown in Fig. 4, the first delay optical path 12a is designed to have an optical path length that is twice as long as the optical path length of the second delay optical path 12b; however, the relationship between the optical path length of the first delay optical path and the optical path length of the second delay optical path may be reversed.

In the individual optical paths 11a, 11b, 12a, and 12b, pairs of lenses 141a and 142b, 142a and 142b, 143a and 143b, and 144a and 144b are provided as relay optical systems that form conjugate surfaces S that are conjugate with the image planes.

In addition, first mirror pair (divergence-angle setting portions) 151a and 151b are provided between the pair of lenses 143a and 143b in the first delay optical path 12a, and second mirror pair (divergence-angle setting portions) 152a and 152b are provided between the pair of lenses 144a and 144b in the second delay optical path 12b. The first mirror pair 151a and 151b fold back the pulsed laser beam that has been reflected by the first beam splitter 13a toward the second beam splitter 13b so as to reach the main optical path 11a by tracing out a rectangular shape. The second mirror pair 152a and 152b fold back the pulsed laser beam that has been reflected by the second beam splitter 13b toward the third beam splitter 13c so as to reach the main optical path 11b by tracing out a rectangular shape.

Here, divergence angles that the individual mirror pair 151a and 151b and mirror pair 152a and 152b give to the pulsed laser beams are determined in accordance with the positions of the individual mirror pair 151a and 151b and mirror pair 152a and 152b in the direction perpendicular to the main optical path 11a and 11b (hereinafter, referred to as Z'-direction), and focal points of the individual pulsed laser beams are formed at different positions in the optical-axis direction depending on the differences among the divergence angles. Specifically, the individual mirror pair 151a and 151b and mirror pair 152a and 152b are disposed at positions that are shifted, in directions perpendicular to the main optical path 11a and 11b, from the reference positions indicated by two-dot chain lines in Fig. 4 at which focal points of all of the pulsed laser beams L1 to L4 are formed at the same position.

By doing so, when the four pulsed laser beams L1 to L4 emitted from the beam splitter apparatus 1 are focused by the objective lens 5b, focal points F1 to F4 are formed at different depths in the specimen A, as shown in Fig. 2.

Intervals ΔFz in the optical-axis direction for the individual focal points F1 to F4 at this time are expressed as ΔFz = ΔZ'/M2 by using displacement levels ΔZ' of the mirror pair 151a and 151b and the mirror pair 152a and 152b from the reference positions, and optical magnifications M achieved at the individual mirror pair 15 and 15b and thereafter.

In this case, with the beam splitter apparatus 1 according to this embodiment, the four pulsed laser beams L1 to L4 that are emitted from the third beam splitter 13c and that are finally radiated onto the specimen A have relative time delays due to the fact that the optical path lengths of the individual optical paths 11a, 11b, 12a, and 12b are different from each other, and are sequentially emitted from the beam splitter apparatus 1 with time intervals that correspond to the optical-path-length differences d therebetween. Specifically, when assuming that the frequency of the pulsed laser beam L0 is Q Hz, the speed of light is C m/s, a delay level d of the second delay optical path 12b is c/4Q m, the frequency of the pulsed laser beams L1 to L4 emitted from the beam splitter apparatus 1 is 4Q Hz, and thus, the repetition frequency of the pulsed laser beams L0 appears to be multiplied.

Here, the optical-path-length differences d are set so that the relative time delays possessed by the pulsed laser beams L1 to L4 become greater than the lifetime of the signal light LS. For example, in the case of observing fluorescence from GFP, which is a typical fluorescent protein, because the lifetime of this fluorescence is about three nanoseconds, the pulsed laser beams L1 to L4 possess relative time delays with respect to each other that are equal to or greater than three nanoseconds.

By shifting the times at which the specimen A is irradiated with the pulsed laser beams L1 to L4 by the amount of time attributed to the relative time delays, it is possible to make the time intervals among the four pulsed laser beams L1 to L4 sufficiently short so that the four pulsed laser beams L1 to L4 can be assumed to be radiated onto the specimen A essentially at the same time while allowing signal lights LS generated at the observation planes P1 to P4 to be detected as signals that are distinct from each other. By doing so, there is an advantage in that it is possible to observe molecular reactions occurring at the four observation planes P1 to P4 having different depths at the same point in time.

Note that, in this embodiment, the individual mirror pair 151a and 151b and mirror pair 152a and 152b may be provided in such a way that they can be moved together in the Z'-direction.

By doing so, it is possible to change the intervals among the focal points F1 to F4 of the pulsed laser beams L1 to L4 in the optical-axis direction, that is, the intervals in the Z-direction among the observation planes P1 to P4. Specifically, it is possible to change the positions of the third observation plane P3 and the fourth observation plane P4 in the Z-direction together by moving the first mirror pair 5a, and it is possible to change the positions of the second observation plane P2 and the fourth observation plane P4 in the Z-direction together by moving the second mirror pair 5b.

In addition, although the beam splitter 13c possessing no polarizing property is employed as a multiplexing portion in this embodiment, alternatively, a half-wave plate may be disposed in the main optical path 11 or the second delay optical path 12b in addition to employing a polarizing beam splitter.

By doing so, it is possible to decrease the amount of light loss in the multiplexing portion.

Next, modifications of the beam splitter apparatus 1 according to this embodiment will be described.

{First Modification}

As shown in Fig. 5, a beam splitter apparatus 1-1 according to a first modification of this embodiment differs from the beam splitter apparatus 1 in that the first delay optical path 12a is provided with another lens pair (relay optical system) 145a and 145b and another mirror pair 153a and 153b that are provided between the lenses of the lens pair 145a and 145b.

The first delay optical path 12a has a rectangular optical path formed via mirrors M1 and M2 and the other mirror pair 153a and 153b. By moving the other mirror pair 153a and 153b together in the direction parallel to the main optical path 11a and 11b, the positions of the focal points F1 to F4 of the individual pulsed laser beams L1 to L4 are changed. The relationship between the amount of movement $X_M$ of the mirror pair 153a and 153b and the amount of movement $X_E$ of the focal points F1 to F4 in the specimen A at this time is expressed by the expression below. Therefore, by employing lenses 145a and 145b having short focal distances, it is possible to decrease the amount of movement of the mirror pair 153a and 153b that is required to change the positions of the focal points F1 to F4.

$$2X_M = X_E (m f_R / f_p)^2$$

Assuming that m is the magnification of the objective lens 5b, d is the working distance of the objective lens 5b, $f_{PB}$ is the back focus of the pupil projection lens 10, fp is the focal distance of the pupil projection lens 10, and $f_R$ is the focal distances of the lenses 145a and 145b in front of and behind the mirror pair 153a and 153b, the relationship below is satisfied:

$$|2X_M| < f_R, \quad f_{PB}(f_R/f_p)^2, \quad d(mf_R/f_p)^2$$

{Second Modification}

As shown in Fig. 6, a beam splitter apparatus 1-2 according to a second modification of this embodiment differs from the beam splitter apparatus 1 in that ND filters (parallel plates) 16 that are disposed in the individual optical paths 11a, 11b, 12a, and 12b are provided as light-intensity adjusting portions that adjust the intensities of the individual pulsed laser beams L1 to L4. The transmittances of the individual ND filters 16 are set so that the intensity becomes the lowest for the pulsed laser beam L1 whose focal point F1 is formed at the shallowest position in the specimen A, and so that the intensity becomes the highest for the pulsed laser beam L4 whose focal point F4 is formed at the deepest position in the specimen A.

In the case in which the specimen A is a scatterer, such as biological tissue, the signal lights LS are subjected to increasing influences of scattering and aberration due to the specimen A with increasing depths of the positions of the observation planes P1 to P4, and thus, the intensities of the signal lights LS detected by the detecting portion 6b decrease. Therefore, by adjusting the intensities of the individual pulsed laser beams L1 to L4 as in this modification, it is possible to compensate for the variability in the detected intensities of the signal lights LS caused by the fact that irradiation positions of the individual pulsed laser beams L1 to L4 differ in the depth direction.

In addition, in the case in which the mirror pair 151a and 151b and the mirror pair 152a and 152b are provided in a movable manner, the intensities of the pulsed laser beams L1 to L4 may be automatically adjusted by moving the ND filters 16 together with the movement of the mirror pair 151a and 151b and that of the mirror pair 152a and 152b by using the ND filters 16 whose transmittances change in a continuous manner. In this case, the relationship between the amounts of movement of the mirror pair 151a and 151b and that of the mirror pair 152a and 152b and the amounts of change in the detected intensities of the signal lights LS should be ascertained by measuring or calculating them in advance.

{Third Modification}

As shown in Fig. 7, a beam splitter apparatus 1 according to a third modification of this embodiment differs from the beam splitter apparatus 1 in that the first beam splitter 13a and the third beam splitter 13c are polarizing beam splitters, that the second beam splitter 13b is a non-polarizing beam splitter, and that half-wave plates (polarization adjusting portions) 17a, 17b, 17c, and 17d are provided in front of the first beam splitter 13a, in the second main optical path 11b, in the first delay optical path 12a, and in the second delay optical path 12b, respectively, so as to serve as light-intensity adjusting portions.

Of the pulsed laser beams that have entered them, the individual polarizing beam splitters 13a and 13c allow P-polarization components to pass therethrough and reflect S-polarization components. Therefore, by adjusting the polarization state of the pulsed laser beam L0 by using the half-wave plate 17a in front of the first polarizing beam splitter 13a, it is possible to change the splitting ratio of the pulsed laser beam at the polarizing beam splitter 13a. The half-wave plate 17c converts the polarization states of the pulsed laser beams that pass through the first delay optical path 12a to P-polarization from S-polarization, thus converting the pulsed light beams that have passed through the second beam splitter 13b so as to be uniformly P-polarized. The half-wave plates 17b and 17d can change transmittances/reflectances of the pulsed laser beams at the polarizing beam splitter 13c by adjusting the polarization states of the pulsed laser beams in the individual optical paths 11b and 12b, which are P-polarized. As a result, it is possible to adjust the intensities of the individual pulsed laser beams L1 to L4.

By doing so also, as with the second modification, it is possible to compensate for the variability in the detected intensities of the signal lights LS caused by the fact that the irradiation positions of the individual pulsed laser beams L1 to L4 differ in the depth direction.

{Fourth Modification}

As shown in Fig. 8, a beam splitter apparatus 1-4 according to a fourth modification of this embodiment differs from the beam splitter apparatus 1 in that positions of the mirror pair 151a and 151b and the mirror pair 152a and 152b are shifted from the reference positions not only in the Z'-direction but also in a direction parallel to the main optical path 11a and 11b (hereinafter, referred to as Y'-direction).

By doing so, as shown in Fig. 9, the focal points F1 to F4 of the individual pulsed laser beams L1 to L4 are positioned differently from each other not only in the optical-axis direction but also in the direction that intersects the optical axis (Y-direction). Therefore, as shown in Fig. 10, it is possible to simultaneously observe the four observation planes P1 to P4 that differ from each other not only in the Z-direction positions but also in the Y-direction positions. Such a configuration is effective for observing signals that are three-dimensionally transmitted, for example, signal transmission of nerves diagonally running in the specimen A. Note that, an interval $\Delta Fy$ among the individual focal points F1 to F4 in the Y-direction at this time is expressed as $\Delta Fy = \Delta Y'/M$ by using a displacement level $\Delta Y'$ of the mirror pair 151a and 151b and that of the mirror pair 152a and 152b from the reference positions in the Y'-direction and optical magnifications M achieved at the individual mirror pair 15 and 15b and thereafter.

Furthermore, by horizontally moving the optical axes of the pulsed laser beams between the mirror pairs in the direction perpendicular to the plane of the figure by changing the angles of the mirror pair 151a and 151b and that of the mirror pair 152a and 152b, it is possible to make the positions of the focal points F1 to F4 of the pulsed laser beams L1 to L4 also differ in the X-direction.

{Fifth Modification}

As shown in Fig. 11, a beam splitter apparatus 1-5 according to a fifth modification of this embodiment differs from the beam splitter apparatus 1 in that the first mirror pair 151a and 151b are shifted from the reference positions in the Z'-direction, and that the second mirror pair 152a and 152b are shifted from the reference positions in the Y'-direction.

By doing so, as shown in Fig. 12, the focal points F1 and F2 of the pulsed laser beams L1 and L2 and the focal points F3 and F4 of the pulsed laser beams L3 and L4 are made to differ from each other in the Z-direction, and the focal points F1 and F3 of the pulsed laser beams L1 and L3 and the focal points F2 and F4 of the pulsed laser beams L2 and L4 are made to differ from each other in the Y-direction. Therefore, as shown in Fig. 13, because it is possible to simultaneously scan each of the two observation planes P1 and P2 whose Z-direction positions are different by using the two pulsed laser beams L1 and L2 or L3 and L4, it is possible to decrease the amount of time required to scan the observation planes P1 and P2 by half.

In this modification, the first mirror pair 151a and 151b may be shifted from the reference positions in the Y'-direction, and the second mirror pair 152a and 152b may be shifted from the reference positions in the Z'-direction. In this case, in Figs. 12 and 13, the pulsed laser beam L2 and the pulsed laser beam L3 are switched.

{Sixth Modification}

As shown in Fig. 14, a beam splitter apparatus 1-6 according to a sixth modification of this embodiment differs from the beam splitter apparatus 1 in that the first mirror pair 151a and 151b are shifted from the reference positions in the Y'-direction and the Z'-direction and that the second mirror pair 152a and 152b are shifted from the reference positions in the Y'-direction.

By doing so, as compared with the positions of the focal points F1 to F4 shown in Fig. 12, the focal points F1 and F2 of the pulsed laser beams L1 and L2 and the focal points F3 and F4 of the pulsed laser beams L3 and L4 are made to differ from each other also in the Y-direction, as shown in Fig. 15. Therefore, as shown in Fig. 16, because it is possible to simultaneously scan each of the two observation planes P1 and P2 whose Z-direction positions and Y-direction positions are different by using the two pulsed laser beams L1 and L2 or L3 and L4, it is possible to decrease the amount of time required to scan the observation planes P1 and P2 by half. Such a configuration is effective in analyzing interactions between two arbitrary points, for example, high-speed signal transmission between two cells or the like.

In this modification, the first mirror pair 151a and 151b may be shifted from the reference positions in the Z'-direction, and the second mirror pair 152a and 152b may be shifted from the reference positions in the Y'-direction and the Z'-direction. In this case, in Figs. 14 and 15, the pulsed laser beam L2 and the pulsed laser beam L3 are switched.

{Second Embodiment}

Next, a beam splitter apparatus 1' according to a second embodiment of the present invention and a laser-scanning microscope provided with the same will be described below with reference to the drawings.

In this embodiment, configurations differing from those of the above-described first embodiment will mainly be described, and configurations common with those of the first embodiment will be given the same reference signs and descriptions thereof will be omitted.

A laser-scanning microscope according to this embodiment is configured in the same manner as the laser-scanning microscope 100 according to the first embodiment.

As shown in Fig. 17, a beam splitter apparatus 1' according to this embodiment is provided with focusing lenses (divergence-angle setting portions) 18a, 18b, and 18c that forms secondary image planes at the rear side of the third beam splitter 13c in the first main optical path 11a, the first delay optical path 12a, and the second delay optical path 12b. In addition, a collimator lens 19 that receives all of the four pulsed laser beams L1 to L4 and that converts the individual beams L1 to L4, which enter from the secondary image planes in the form of diverging beams, to substantially collimated beams is provided behind the third beam splitter 13c.

Here, the individual focusing lenses 18a to 18c are disposed at positions that are shifted, in the respective optical-axis directions, from the reference positions at which focal points of all of the pulsed laser beams L1 to L4 are formed at the same position. By doing so, as with the first embodiment, when the four pulsed laser beams L1 to L4 emitted from the beam splitter apparatus 1 are focused by the objective lens 5b, focal points are formed at different depths in the specimen A, as shown in Fig. 2, and the four pulsed laser beams L1 to L4 are substantially simultaneously scanned in a synchronized manner on the four observation planes P1 to P4 at different depth positions in the specimen A. Therefore, it is possible to afford the same advantages as those of the first embodiment.

Note that, in this embodiment, the individual focusing lenses 18a to 18c may be provided so as to be movable in the optical-axis directions of the individual optical paths 11a, 12a, and 12b. By doing so, it is possible to change the intervals in the Z-direction among the individual observation planes P1 to P4 by changing the intervals in the Z-direction among the focal points F1 to F4 of the individual pulsed laser beams L1 to L4.

Specifically, it is possible to change the positions of the first observation plane P1 and the second observation plane P2 in the Z-direction together by moving the focusing lens 18a, it is possible to change the positions of the third observation plane P3 and the fourth observation plane P4 in the Z-direction together by moving the focusing lens 18b, and it is possible to change the positions of the second observation plane P2 and the fourth observation plane P4 in the Z-direction together by moving the focusing lens 18c. At this time, because the delay times possessed by the individual pulsed laser beams L1 to L4 do not fluctuate in association with the movement of the focusing lenses 18a to 18c, there is an advantage in that time control can easily be performed.

In addition, in this embodiment, although Fig. 17 shows an optical-path design in which the first delay optical path 12a has an optical path length that is twice as long as the optical path length of the second delay optical path 12b, the relationship between the optical path length of the first delay optical path and the optical path length of the second delay optical path may be reversed.

In addition, as the third beam splitter 13c of the multiplexing portion in this embodiment, a half-wave plate may be disposed in the second delay optical path 12b in addition to employing a polarizing beam splitter. By doing so, it is possible to decrease the amount of light loss in the multiplexing portion.

In addition, this embodiment may be combined with the configuration described in the first embodiment in which divergence angles are given to the individual pulsed laser beams L1 to L4 by adjusting the positions of the individual mirror pair 151a and 151b and mirror pair 152a and 152b.

In addition, in this embodiment, as in the second modification and the third modification of the first embodiment, the intensities of the individual pulsed laser beams L1 to L4 may be adjusted by using the ND filters disposed in the individual optical paths 11a, 11b, 12a, and 12b or by utilizing a combination of the polarizing beam splitters and the half-wave plates.

Next, a modification of the beam splitter apparatus 1' according to this embodiment will be described.

A beam splitter apparatus according to a modification of this embodiment differs from the beam splitter apparatus 1' in that the focusing lens 18a and the focusing lens 18b are provided so as to be movable along the optical axes in synchronization with each other by means of motors (not shown).

The first observation plane P1 and the second observation plane P2 are moved in the Z-direction by moving the focusing lens 18a, and the third observation plane P3 and the fourth observation plane P4 are moved in the Z-direction by moving the focusing lens 18b. Therefore, by moving these two focusing lenses 18a and 18b in a synchronized manner, it is possible to move all of the observation planes P1 to P4 together in the Z-direction. By doing so, it is possible to acquire a three-dimensional image of the specimen A at high speed by scanning the four pulsed laser beams L1 to L4 at high speed not only in the X-direction and the Y-direction but also in the Z-direction.

Note that in the first and second embodiments, another set of the first to third beam splitters 13a to 13c may be connected in series behind the first to third beam splitters 13a to 13c, and another set of the main optical path 11a and 11b and the delay optical paths 12a and 12b described above may be formed.

By doing so, the number of pulsed laser beams to be generated from the single pulsed laser beam L0 by using the beam splitter apparatus 1 or 1' can be increased from four to 16, 64, and so on.

Note that, although the first and second embodiments have been described by using a laser-scanning microscope as an example, the beam splitter apparatuses and the scanning observation apparatuses of the present invention can also be applied to a laser-scanning endoscope. Specifically, by providing an observation optical system 5 and a wave guiding path (for example, an optical fiber) that receives the signal lights LS and transmits them to the detection system 6 at the distal-end portion of an inserted portion provided in the laser-scanning endoscope, it suffices to supply the pulsed laser beams L1 to L4 to the observation optical system 5, via an optical fiber or the like, from the beam splitter apparatus 1 or 1' disposed at the basal end of the inserted portion.

{Reference Signs List}

1, 1' beam splitter apparatus
2 laser light source
3 scanning portion
4 stage
5 observation optical system
5a imaging lens
5b objective lens
6 detection system
6b detecting portion
7 control portion
8 restoring portion
9 display portion
10 pupil projection lens
11a, 11b main optical path
12a, 12b delay optical path (delaying portion)
13a, 13b beam splitter (demultiplexing portion)
13c beam splitter (multiplexing portion)
141a to 145a, 141b to 145b lens (relay optical system)
151a to 153a, 151b to 153b mirror pair (divergence-angle setting portion)
16 ND filter (parallel plate)
17a, 17b, 17c, 17d half-wave plate
18a, 18b, 18c focusing lens (divergence-angle setting portion)
19 collimator lens
100 laser-scanning microscope

**Claims**

1. A beam splitter apparatus that is applied to an observation apparatus that irradiates a specimen with pulsed light beams to induce a molecular reaction in the specimen and that observes signal lights generated by this reaction, the beam splitter apparatus comprising:

   a demultiplexing portion that splits an input pulsed light beam into a plurality of optical paths;
   relay optical systems that are provided in the plurality of optical paths and that relay the pulsed light beams guided through the individual optical paths;
   a multiplexing portion that multiplexes the plurality of the pulsed light beams that have been relayed through the individual optical paths by the relay optical systems;
   delaying portions that are provided in the plurality of optical paths and that give the pulsed light beams, which are guided through the individual optical paths, relative time delays that are large enough to separate the plurality of the signal lights from each other; and
   divergence-angle setting portions that are provided in the plurality of optical paths and that give the pulsed light beams that are guided through the individual optical paths divergence angles that are different from each other.

2. The beam splitter apparatus according to Claim 1,
   wherein the relay optical systems are provided with pairs of lenses, and
   each of the divergence-angle setting portions is disposed between the pair of lenses.

3. The beam splitter apparatus according to Claim 2,
   wherein the divergence-angle setting portions are provided with mirror pairs that deflect the pulsed light beams split by the demultiplexing portion toward the multiplexing portion so as to trace out a rectangular shape, and
   the mirror pairs are provided in such a way that the mirror pairs can be moved together in a direction parallel to entrance optical axes of the pulsed light beams coming from the demultiplexing portion.

4. The beam splitter apparatus according to Claim 3, wherein the mirror pairs are provided in such a way that the mirror pairs can be moved together in a direction that intersects the entrance optical axes of the pulsed light beams coming from the demultiplexing portion.

5. The beam splitter apparatus according to any one of Claims 1 to 4, wherein the divergence-angle setting portions are provided with

focusing lenses that are provided in the plurality of optical paths and that convert the pulsed light beams that are guided through the individual optical paths to converging beams that are converged at positions differing from each other in the optical-axis direction; and

a collimator lens that is provided behind the multiplexing portion and that receives all of the pulsed light beams that have been converted to the converging beams by the individual focusing lenses and that converts the received beams to substantially collimated beams having different divergence angles from each other.

6. The beam splitter apparatus according to Claim 5, wherein the individual focusing lenses are provided so as to be movable in the optical-axis direction.

7. The beam splitter apparatus according to Claim 5 or 6, wherein the individual focusing lenses are provided so as to be movable in the optical-axis direction in synchronization with each other.

8. The beam splitter apparatus according to any one of Claims 1 to 7, further comprising:

light-intensity adjusting portions that are provided in the plurality of optical paths and that adjust intensities of the pulsed light beams that are guided along the individual optical paths.

9. The beam splitter apparatus according to Claim 8,

wherein at least one of the demultiplexing portion and the multiplexing portion is provided with a polarizing beam splitter, and

the light-intensity adjusting portions are provided with a polarization adjusting portion that is provided in front of the polarizing beam splitter and that adjusts the polarization of the pulsed light beams.

10. The beam splitter apparatus according to Claim 8, wherein the light-intensity adjusting portions are provided with parallel plates that are provided in the plurality of optical paths and with which transmittances of the pulsed light beams can be changed.

11. The beam splitter apparatus according to any one of Claims 1 to 10, wherein multiple sets of the demultiplexing portion, the relay optical systems, the multiplexing portion, the delaying portions, and the divergence-angle setting portions are provided in series.

12. A scanning observation apparatus comprising:

the beam splitter apparatus according to any one of Claims 1 to 11;

a scanning portion that scans a plurality of pulsed light beams emitted from the beam splitter apparatus in a direction that intersects the optical axes;

an observation optical system that irradiates the specimen with the pulsed light beams scanned by the scanning portion; and

a detection system that detects the signal lights coming from the specimen.

13. The scanning observation apparatus according to Claim 12, wherein the observation optical system is provided with an objective lens that focuses the pulsed light beams on the specimen.

14. The scanning observation apparatus according to Claim 12 or 13, further comprising:

a control portion that synchronizes a timing at which the detection system detects the signal lights with the pulsed light beams;

a restoring portion that restores two-dimensional information or three-dimensional information by associating the signal lights detected by the detection system and the scanning positions of the pulsed light beams on the specimen; and

a display portion that displays the two-dimensional information or the three-dimensional information that has been restored by the restoring portion.

15. A laser-scanning microscope comprising:

the scanning observation apparatus according to any one of Claims 12 to 14; and
a laser light source that supplies the beam splitter apparatus with pulsed laser beams that serve as the pulsed light beams.

16. A laser-scanning endoscope comprising:

the scanning observation apparatus according to any one of Claims 12 to 14.

# FIG. 1

EP 2 960 703 A1

# FIG. 2

# FIG. 3

# FIG. 4

# FIG. 5

# FIG. 6

# FIG. 7

# FIG. 8

## FIG. 9

## FIG. 10

FIG. 11

# FIG. 12

# FIG. 13

# FIG. 14

# FIG. 15

# FIG. 16

FIG. 17

EP 2 960 703 A1

## INTERNATIONAL SEARCH REPORT

| International application No. |
| --- |
| PCT/JP2014/054513 |

### A. CLASSIFICATION OF SUBJECT MATTER
*G02B21/06*(2006.01)i, *A61B1/00*(2006.01)i, *G02B23/24*(2006.01)i

According to International Patent Classification (IPC) or to both national classification and IPC

### B. FIELDS SEARCHED

Minimum documentation searched (classification system followed by classification symbols)
G02B21/06, A61B1/00, G02B23/24

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X<br>Y | WO 2011/052248 A1 (OLYMPUS CORP.),<br>05 May 2011 (05.05.2011),<br>paragraphs [0040] to [0082], [0116] to [0144],<br>[0174] to [0189]; fig. 6 to 8<br>& JP 2013-509609 A  & US 2012/0271111 A1<br>& CN 102597845 A | 1-2<br>3-16 |
| X<br>Y | JP 2011-197609 A (Olympus Corp.),<br>06 October 2011 (06.10.2011),<br>paragraphs [0008] to [0023], [0026] to [0065];<br>fig. 1, 7, 8<br>(Family: none) | 1-2<br>3-16 |
| Y | JP 2005-31589 A (Olympus Corp.),<br>03 February 2005 (03.02.2005),<br>paragraph [0019]; fig. 5<br>(Family: none) | 3-16 |

☒ Further documents are listed in the continuation of Box C.   ☐ See patent family annex.

| | |
| --- | --- |
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
| --- | --- |
| Date of the actual completion of the international search<br>   28 March, 2014 (28.03.14) | Date of mailing of the international search report<br>   08 April, 2014 (08.04.14) |
| Name and mailing address of the ISA/<br>   Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2014/054513

C (Continuation).  DOCUMENTS CONSIDERED TO BE RELEVANT

| Category* | Citation of document, with indication, where appropriate, of the relevant passages | Relevant to claim No. |
|---|---|---|
| A | JP 5-93845 A  (Olympus Optical Co., Ltd.), 16 April 1993 (16.04.1993), paragraph [0016]; fig. 2 & US 5245173 A        & EP 531543 A1 & WO 1992/017805 A1      & DE 69231269 T2 & AT 194871 T | 1-16 |

Form PCT/ISA/210 (continuation of second sheet) (July 2009)

**EP 2 960 703 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004109219 A